# FASCICULE DE BREVET EUROPEEN

(11) **EP 4 508 409 B1**
(45) Date de publication et mention de la délivrance du brevet: **18.03.2026**
(21) Numéro de dépôt: 23722436.5
(22) Date de dépôt: 13.04.2023
(51) Int. Cl.: G01N 1/28, G01N 1/30, G01N 15/05, G01N 21/27, G01N 33/49

(54) **DISPOSITIF D'ÉTALEMENT OU COLORATION ET DE DÉTERMINATION DE VITESSE DE SÉDIMENTATION**
VORRICHTUNG ZUM STREUEN ODER ANFÄRBEN UND ZUM BESTIMMEN EINER SEDIMENTATIONSRATE
DEVICE FOR SPREADING OR STAINING AND FOR DETERMINING A SEDIMENTATION RATE

(30) Priorité: 15.04.2022 FR 2203573
(43) Date de publication de la demande: 19.02.2025
(73) Titulaire: Horiba ABX SAS, 34790 Grabels (FR)
(72) Inventeur: COUDERC, Guilhem, 34184 MONTPELLIER Cedex (FR); BEAUDUCEL, Florent, 34184 MONTPELLIER Cedex (FR); THORAVAL, Coralie, 34184 MONTPELLIER Cedex (FR)
(74) Mandataire: Plasseraud IP
(86) Numéro de dépôt international: PCT/FR2023/050536
(87) Numéro de publication internationale: WO 2023/199001

(56) Documents cités:
- EP-A1- 2 921 862
- WO-A1-2021/097610
- IT-A1- UA20 163 693

## Description

L'invention concerne le domaine de l'hématologie, et plus particulièrement la détermination de la vitesse de sédimentation (ci-après VS, également appelée "erythrocyte sedimentation rate" ou "ESR" en anglais).

La vitesse de sédimentation fait partie des examens de routine avec la numération formule sanguine (ci-après NFS ou CBC pour "Complete Blood Count" en anglais) effectuée au cours d'un bilan sanguin et permet de détecter la présence de pathologies inflammatoires ou infectieuses. Par exemple un rhumatisme, un cancer et d'autres affections entraînant des changements de la concentration en protéines dans le sang. Une VS élevée indique un état inflammatoire sans préjuger de sa nature. Il arrive cependant que la VS reste basse alors qu'il y a un syndrome inflammatoire. Une VS élevée indique donc généralement la présence d'une pathologie. L'association de ce test non spécifique avec des examens complémentaires permet d'avoir un diagnostic plus précis.

La méthode de référence de la mesure de la VS est la méthode Westergren. Un tube étroit de dimensions normalisées est rempli d'un échantillon de sang prélevé avec un anticoagulant, par exemple de l'EDTA, et dilué au citrate de sodium et placé verticalement. La mesure de VS est la hauteur, en millimètres, de la colonne de plasma après une heure de sédimentation. Par exemple, il est considéré qu'une hauteur inférieure à 10 mm après la première heure de sédimentation est une valeur normale pour un adulte masculin. La limite de normalité n'est pas une valeur absolue mais dépend de l'âge et du sexe du patient.

La VS est la résultante de trois étapes : agrégation des hématies ou globules rouges, sédimentation, et tassement des rouleaux.

Le phénomène d'agrégation des hématies est produit en particulier par les protéines du sang avec une force d'autant plus grande que les concentrations de ces dernières sont élevées. La formation des agrégats est l'empilement des hématies en "rouleaux", puis en structures tridimensionnelles. La vitesse de sédimentation dépend des dimensions des agrégats et de la viscosité du plasma.

Pendant les dernières étapes, les agrégats vont sédimenter, c'est-à-dire qu'ils vont progressivement tomber au fond du tube, puis se compacter, ce qui va avoir pour effet de séparer le prélèvement en une portion translucide claire (plasma), en haut, et une portion beaucoup plus foncée (hématies) en bas. La VS est mesurée par la hauteur de la portion translucide.

Le paramètre inflammatoire le plus pertinent est le phénomène d'agrégation qui est directement dépendant des concentrations des protéines plasmatiques. C'est ce phénomène qui est mesuré par la présente invention pour retourner une valeur de VS. C'est également un paramètre non spécifique, mais qui supprime des interférences résultantes des deux dernières étapes de la VS. Il n'y a pas de paramètre normalisé permettant d'exprimer cette vitesse ou dynamique/cinétique d'agrégation. Il est donc pertinent de transposer des grandeurs de mesure d'agrégation en VS.

De par la quantité de sang (1,6 ml) et la durée nécessaire à sa réalisation (1 heure), la méthode Westergren n'est pas compatible avec les automates d'hématologie pour l'analyse CBC. Pour contourner ces problèmes, des recherches ont été menées afin d'utiliser des mesures optiques d'extinction (absorption et diffusion) pour déterminer la vitesse de sédimentation, comme dans le brevet US 6 632 679.

Certains brevets, comme décrit dans EP 2 921 862 ont proposé d'intégrer une mesure de VS modifiée dans un appareil de test sanguin qui prélève une quantité de sang et la sépare en deux portions qui font respectivement l'objet d'un comptage (NFS) et d'une mesure de vitesse de sédimentation.

Cet appareil lie donc la mesure de comptage et celle de mesure de vitesse de sédimentation, ce qui est désavantageux car nécessite un appareillage complexe pour distribuer via un unique prélèvement, deux portions d'échantillons de sang vers la section de comptage d'un côté et vers la section de mesure de vitesse de sédimentation d'un autre, d'autant plus que les opérations CBC et de mesure de VS ne présentent pas les mêmes besoins en termes de préparation de l'échantillon.

D'une manière plus générale, dans les laboratoires automatisés de type TLA ("Total Laboratory Automation" en anglais), le flux de tube est géré de manière totalement automatique, des tests les plus fréquents à ceux les moins fréquents. Le matériel nécessaire pour alimenter en échantillon la cellule de mesure est de loin la partie la plus coûteuse et complexe, et comprend :
- l'interconnexion à une chaîne,
- la gestion de racks,
- l'agitation des tubes,
- la gestion des tubes,
- le prélèvement et transfert dans la cellule de mesure,
- le rinçage du dispositif de prélèvement, et
- les dispositifs de contrôle et de transmission des résultats.

Aussi, il en ressort un ratio très élevé (supérieur à 100) entre le coût du système de mesure et celui de l'infrastructure nécessaire pour gérer l'échantillon. En comparaison, ce ratio est beaucoup plus faible (2 à 5) pour un système de mesure CBC/DIF/RET ou un système d'étalement ou coloration automatisé couramment utilisé dans un laboratoire d'hématologie.

D'autre part, les laboratoires traitant un grand nombre de tubes quotidiennement exigent que le temps de traitement des tubes nouvellement arrivés soit limité (en anglais "Turn around time" ou TAT) et cela contraint directement le dimensionnement des systèmes de mesure CBC/DIF/RET, et donc leur coût matériel.

Par contre, comme une faible proportion des tubes traités nécessite un étalement ou une coloration (seulement 5 à 20%), ce système est couramment surdimensionné chez tous les fabricants.

Le document WO 2021/097610 A1 décrit un analyseur d'échantillons comprenant un module de mesure de la vitesse de sédimentation des érythrocytes, un module de mesure de l'hémogramme et un module d'attribution d'échantillons. Le module d'attribution d'échantillons est utilisé pour collecter un échantillon de sang, attribuer une première partie de l'échantillon de sang au module de mesure de la vitesse de sédimentation des érythrocytes et d'attribuer une deuxième partie de l'échantillon sanguin au module de mesure de la numération sanguine complète. Le module de mesure de la vitesse de sédimentation des érythrocytes comprend un tube de mesure et un appareil de mesure optique.

Aucun dispositif connu ne permet de réaliser efficacement une détermination de la mesure de vitesse de sédimentation de manière rapide et intégrée dans le contexte des exigences d'un laboratoire moderne.

L'invention vient améliorer la situation. À cet effet, elle propose un dispositif d'étalement ou coloration et de détermination de vitesse de sédimentation qui comprend un premier groupe agencé pour prélever un échantillon sanguin dans un tube et pour réaliser un frottis sur cet échantillon, et un deuxième groupe agencé pour prélever un échantillon sanguin dans un tube et pour réaliser une détermination de vitesse de sédimentation. Le dispositif comprend au moins un organe de prélèvement pouvant être commandé pour une opération par le premier groupe et une opération par le deuxième groupe pour le prélèvement d'un échantillon sanguin de sorte qu'un échantillon prélevé pour le premier groupe n'est pas utilisé par le deuxième groupe, et qu'un échantillon prélevé pour le deuxième groupe n'est pas utilisé par le premier groupe, le deuxième groupe étant muni d'un capteur comprenant une source de lumière à infrarouge et un capteur optique disposés sensiblement en regard l'un de l'autre autour d'un tube relié à une extrémité de sortie de l'au moins un organe de prélèvement de sorte que la lumière émise par la source de lumière infrarouge atteint le capteur optique après avoir traversé ledit tube. Le deuxième groupe est en outre agencé pour réaliser un rinçage de l'organe de prélèvement et du tube entre deux déterminations de mesures de vitesse de sédimentation et le capteur optique est agencé pour réaliser une mesure de blanc après une opération de rinçage. Le dispositif comprend en outre un convertisseur agencé pour recevoir une mesure de blanc et une ou plusieurs mesures de transmission lumineuse du capteur optique et pour déterminer une vitesse de sédimentation à partir du rapport entre la mesure de blanc et la ou les plusieurs mesures de transmission lumineuse.

Ce dispositif est particulièrement avantageux car il permet de réaliser la mesure de vitesse de sédimentation indépendamment de la numération de formule sanguine. Ainsi, la mesure de vitesse de sédimentation, qui reste un test moins systématique, ne vient pas interférer avec l'architecture du dispositif d'une manière susceptible de pénaliser les autres fonctionnalités.

Selon divers modes de réalisation, l'invention peut présenter une ou plusieurs des caractéristiques suivantes :
- la source de lumière à infrarouge et le capteur optique sont disposés à une distance de l'extrémité de prélèvement de l'organe de prélèvement inférieure à 10cm,
- le deuxième groupe est disposé à une entrée du dispositif en amont du premier groupe, ou en sortie du dispositif pour la réalisation de mesures complémentaires de type étalement ou coloration en aval du premier groupe,
- le convertisseur est agencé pour déterminer un instant de mesure de transmission lumineuse la plus basse, et un instant de mesure de transmission lumineuse final,
- le capteur optique est agencé pour mettre en œuvre un gain maximal entre l'instant de mesure de transmission lumineuse la plus basse et l'instant de mesure de transmission lumineuse final, et pour mettre en œuvre un gain minimal le reste du temps,
- le convertisseur est agencé pour calculer la vitesse de sédimentation à partir du ratio entre d'une part le rapport entre la mesure de blanc et la mesure à l'instant de mesure de transmission final, et d'autre part le rapport entre la mesure de blanc et la mesure à l'instant de mesure de transmission lumineuse de transmission lumineuse la plus basse,
- le capteur optique est commandé avec un gain faible avant que du sang passe dans la portion sensiblement transparente, et avec un gain fort après, et
- l'organe de prélèvement est une aiguille pouvant être commandée pour le prélèvement d'un échantillon sanguin à laquelle est relié un tube dans lequel est réalisée la portion sensiblement transparente.

L'invention concerne également un procédé d'étalement ou coloration et de détermination de vitesse de sédimentation caractérisé en ce qu'il comprend l'utilisation d'un dispositif selon l'invention, et en ce que la réalisation d'un étalement ou coloration d'une part et la détermination d'une vitesse de sédimentation d'autre part comprennent le prélèvement de deux échantillons distincts.

D'autres caractéristiques et avantages de l'invention apparaîtront mieux à la lecture de la description qui suit, tirée d'exemples donnés à titre illustratif et non limitatif, tirés des dessins sur lesquels :
- la figure 1 représente une vue schématique générale d'un dispositif selon l'invention,
- la figure 2 représente un détail de réalisation d'un élément de la figure 1,
- la figure 3 représente un diagramme de mesure par le dispositif de la figure 1, et
- la figure 4 représente une vue schématique d'un mode de réalisation du dispositif de la figure 1.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

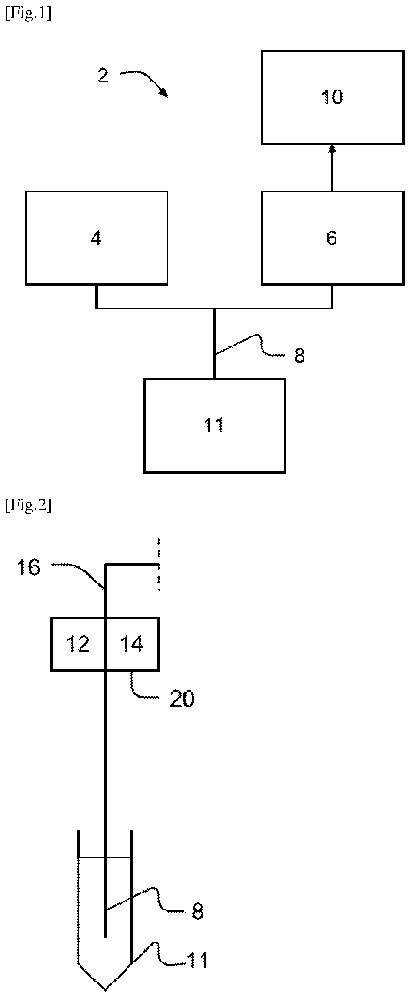

La figure 1 représente une vue schématique générale d'un dispositif 2 de numération de formule sanguine et de détermination de la mesure de vitesse de sédimentation selon l'invention. Le dispositif 2 comprend un premier groupe 4, un deuxième groupe 6, un organe de prélèvement 8 et un convertisseur 10.

Le premier groupe 4 est agencé pour réaliser un frottis sanguin. Des dispositifs réalisant des frottis sanguins de manière automatique sont très couramment mis en œuvre dans les laboratoires ayant à traiter un grand nombre de tubes quotidiennement. Ils sont généralement largement sur dimensionnés en cadence de traitement au regard des besoins du laboratoire.

Ce surdimensionnement est directement lié à la complexité technique des différentes étapes nécessaires à la préparation de la lame de chacun des dispositifs (prélèvement, dépôt d'une goutte d'aliquote sur une lame de verre, un étalement, une identification de la lame, des colorations vitales...) : la cadence élevée constitue le seul moyen pour les fabricants d'en optimiser les coûts marginaux.

Les frottis sanguins étant des tests moins systématiques que le CBC, la Demanderesse a découvert qu'il était intéressant de mutualiser les ressources du premier groupe 4 pour ajouter une détermination de la mesure de l'ESR au moyen du groupe 6.

Le deuxième groupe 6 est agencé pour déterminer une mesure de VS. C'est ce deuxième groupe qui fait l'objet principal de l'invention. En effet, comme décrit en introduction, ce test, qu'il soit réalisé selon la méthode classique ou par mesure de changement de l'absorbance lumineuse, est soit fastidieux, soit couplé à une mesure CBC, qui ralentit l'ensemble du dispositif.

La mise en œuvre du deuxième groupe 6 selon l'invention permet d'extrapoler une mesure de vitesse de sédimentation par changement de l'absorbance lumineuse d'une manière découplée de toute mesure CBC ou autres mesures sanguines. Pour cela, le deuxième groupe 6 est agencé pour commander l'organe de prélèvement 8 qui est ici une aiguille indépendamment du premier groupe 4, c'est-à-dire que les échantillons prélevés par le premier groupe 4 ne sont utilisés que pour réaliser un frottis sanguin, et les échantillons prélevés par le deuxième groupe 6 ne sont utilisés que pour une mesure ESR.

Ainsi, comme on peut le voir sur la figure 1, le premier groupe 4 ou le deuxième groupe 6 commande l'aiguille 8 pour prélever un échantillon sanguin dans un tube de sang 11. Ensuite, cet échantillon est amené au premier groupe 4 pour réaliser un frottis sanguin, ou au deuxième groupe 6 pour réaliser une mesure de changement de l'absorbance lumineuse. Dans ce dernier cas, la mesure est transmise au convertisseur 10 qui retourne un signal ESR et/ou une valeur de mesure de VS.

La figure 2 représente un exemple de réalisation du deuxième groupe de mesure 6. Selon cet exemple, le deuxième groupe de mesure 6 est réalisé au moyen d'une source optique 12 et d'un capteur optique 14 qui sont assemblés en regard l'un de l'autre autour d'un tube 16 qui sert à transporter l'échantillon de sang prélevé par l'aiguille 8 depuis un tube de sang 11 vers l'intérieur du dispositif 2.

Dans l'exemple décrit ici, la source lumineuse 12 est de type LED à longueur d'onde infrarouge de manière préférée dans la plage 700-980 nm et en particulier 800 nm, qui est un point isobestique entre l'oxyhémoglobine et la désoxyhémoglobine ce qui rend la mesure insensible au niveau de la saturation du sang en oxygène. Dans l'exemple décrit ici, le capteur optique 14 est de type photodiode et peut être choisi parmi les capteurs PMT, PDA, CMOS, etc... Dans l'exemple décrit ici, le tube 16 est en téflon et est raccordé à l'aiguille 8. En variante, le tube 16 peut être en verre ou plastique et doit être choisi de manière à offrir une bonne transparence à la longueur d'onde de la source lumineuse 12.

L'ensemble source lumineuse 12 et capteur optique 14 peut être vu comme un unique capteur 20 du deuxième groupe 6. Comme dans l'exemple décrit ici, il peut être en deux parties qui s'assemblent pour enserrer l'aiguille 8 (par exemple métallique) et le tube 16. En variante, la source lumineuse 12 et le capteur optique 14 peuvent être réalisés d'un seul tenant. De manière préférée, le capteur 20 est disposé assez proche de l'extrémité de l'aiguille 8, à moins de 10cm de celle-ci, afin d'optimiser le cisaillement des globules rouges. De manière préférée, cette distance est d'environ 5cm pour obtenir les meilleurs résultats. Dans une variante le capteur 20 peut être placé directement en sortie de l'aiguille 8.

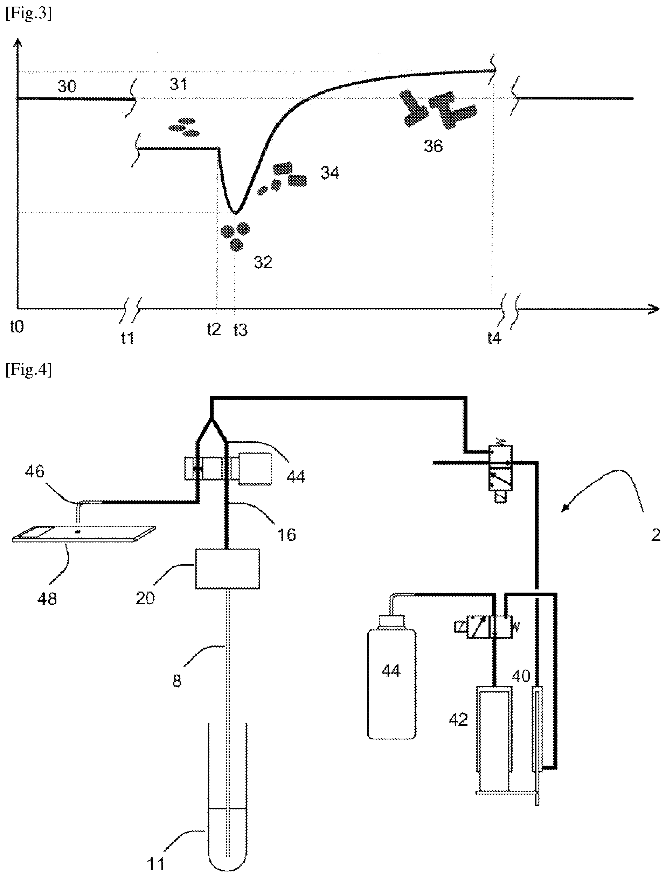

Quelle que soit la configuration, le deuxième groupe 6 permet d'utiliser de manière avantageuse le diamètre de l'aiguille 8 qui est classiquement de l'ordre de 1mm. En effet, lors d'un prélèvement par l'aiguille 8, les globules rouges subissent des déformations successives qui sont décrites ci-après et qui sont représentées sur la figure 3.

Ainsi, une fois l'aiguille 8 plongée dans le tube de sang 11, le deuxième groupe 6 commande une aspiration par l'aiguille 8 déjà amorcée en diluant afin de prélever du sang. Cela est réalisé grâce à des moyens de contrôle d'automatisme, de bacs de préparation et d'électrovannes et seringues connus par ailleurs et non représentés pour des raisons de simplification. Ces moyens entraînent le déplacement du diluant qui occupe le tube 16. A cette étape, du fait de la nature translucide du tube 16 et du diluant qu'il contient, le capteur 20 mesure un signal optique constant.

Ensuite, l'aliquote de sang est déplacée jusqu'au capteur 20 par aspiration. Les globules rouges subissent un cisaillement, ce qui casse l'agrégation. À cette étape, le signal optique reste maximal puisque le sang n'est pas encore en regard du capteur 20. Sur la figure 3, cela est représenté avec la référence 30.

Lorsque le sang arrive dans le capteur, le signal optique représente l'état cisaillé du sang. À ce stade de cisaillement du sang, les hématies ont une forme allongée, comme visible avec la référence 31.

Lorsque l'aspiration s'arrête afin de stopper le cisaillement, le signal de transmission optique baisse du fait que les globules rouges reprennent leur forme relaxée de disque biconcave. Sur la figure 3, cela est représenté avec la référence 32. Ensuite, le signal optique augmente suivant une progression pseudo logarithmique. L'augmentation progressive du signal optique mesurée par le capteur optique 14 est liée à l'agrégation progressive des globules rouges libres. L'agrégation se fait par l'empilement des globules rouges formant des rouleaux puis des structures en trois dimensions ce qui est représenté sur la figure 3 avec les références 34 et 36.

Au-delà d'environ 40s selon les sangs, l'agrégation est fortement ralentie et la sédimentation commence. Ce début de sédimentation interfère sur la mesure d'intérêt qui est l'agrégation et de sa corrélation avec la vitesse de sédimentation. Après t4 l'aliquote de sang est évacuée, par exemple par déplacement de l'aiguille 8 au-dessus d'un bac. On procède ainsi à l'évacuation du sang et au rinçage du tube 16 avec du diluant, le tube 16 se trouvant à la fin de cette étape entièrement rempli de diluant comme au début de la procédure décrite plus haut.

Quatre instants sont représentés sur la figure 3 :
- avant l'instant t1, le tube 16 est rempli de diluant,
- l'instant t1 marque l'instant auquel le sang arrive au niveau du capteur 20 par aspiration,
- l'instant t2 marque l'instant auquel l'aspiration cesse,
- l'instant t3 marque l'instant à partir duquel les globules rouges ont repris leur forme relaxée, et l'agrégation commence,
- l'instant t4 marque l'instant de fin de la procédure, avec l'évacuation du sang qui est à nouveau remplacé par du diluant, comme avant l'instant t1.

L'observation de la figure 3 montre que le signal optique est moins important après l'instant t4, alors que le tube 16 est rempli de diluant, que lorsque le tube 16 est rempli de sang, ce qui peut paraître paradoxal. Cela s'explique par le fait que dans l'exemple décrit ici le capteur optique 14 est conditionné de manière différente avant l'instant t1 et après l'instant t4 de la figure 3.

Ainsi, avant l'instant t1 et après l'instant t4, il est déterminé que la mesure correspond à une mesure de "blanc". Pour toute cette période, le capteur optique 14 est conditionné par le convertisseur 10 avec un gain de mesure minimal.

L'instant t3 correspond au point le plus bas de la période t1-t4, à partir duquel le signal optique devient croissant, ce qui marque, comme décrit plus haut, le début du phénomène que l'invention vise à mesurer. Pour cette raison, et comme la variation du signal optique reste faible entre l'instant t0 et l'instant t1, dans l'exemple décrit ici, le capteur optique 14 est conditionné avec un gain minimal jusqu' à l'instant t1, puis avec un gain maximal entre l'instant t1 et t4, puis à nouveau avec un gain minimal après l'instant t4 pour la mesure suivante.

Cela est d'autant plus avantageux que, dans le mode de réalisation décrit ici, le convertisseur 10 est agencé pour déterminer une mesure basée sur la densité optique du signal mesuré par le capteur optique 14. Pour rappel, la densité optique est définie par la formule *DO*(*t*) = *log*(*KI0*/*I*(*t*)) où I(t) est la mesure du capteur optique à l'instant t et K le rapport gain maxi/gain mini. Plus précisément, le convertisseur 10 est agencé pour retourner une mesure de vitesse de sédimentation basée sur le rapport DO(t3)/DO(t4). La Demanderesse a réalisé de nombreuses mesures de vitesse de sédimentation en utilisant la méthode de référence Westergren qui permettent au convertisseur 10 d'associer les mesures ainsi calculées à une valeur de VS.

La Demanderesse a découvert qu'il est particulièrement avantageux d'utiliser la densité optique, qui permet de ne pas dépendre d'éventuelles variations dans la mesure de transmittance. La Demanderesse a également découvert que le convertisseur 10 peut aussi fonctionner à partir du rapport I(t₄)/I(t₃), sans passer par la densité optique au sens de Beer-Lambert et en utilisant I0 différemment dans le calcul.

Le convertisseur 10 peut être réalisé de diverses manières, par exemple sous la forme d'un code informatique approprié exécuté sur un ou plusieurs processeurs. Par processeurs, il doit être compris tout processeur adapté aux calculs décrits plus bas. Un tel processeur peut être réalisé de toute manière connue, sous la forme d'un microprocesseur pour ordinateur personnel, portable, tablette ou smartphone, d'une puce dédiée de type FPGA ou SoC, d'une ressource de calcul sur une grille ou dans le cloud, d'une grappe de processeurs graphiques (GPUs), d'un microcontrôleur, ou de toute autre forme propre à fournir la puissance de calcul nécessaire à la réalisation décrite plus bas. Un ou plusieurs de ces éléments peuvent également être réalisés sous la forme de circuits électroniques spécialisés tel un ASIC. Une combinaison de processeur et de circuits électroniques peut également être envisagée. Dans le cas de l'unité d'apprentissage automatique basé sur renforcement du gradient, des processeurs dédiés à l'apprentissage automatique pourront aussi être envisagés. En variante, le convertisseur 10 peut être un calculateur analogique sans programmation ou code informatique à proprement parler.

Toujours en variante, le convertisseur 10 pourrait utiliser un algorithme d'apprentissage automatique ("machine learning" en anglais), impliquant ou non un réseau de neurones (profond ou pas). Il s'agit d'associer les mesures d'intensité du capteur optique 14 avec une valeur de vitesse de sédimentation. Cette variante peut être particulièrement utile pour se passer de la densité optique. Toujours en variante, le gain du capteur optique 14 pourrait être le même pour tous les instants de la mesure.

La figure 4 représente une vue schématique d'un mode de réalisation du dispositif de la figure 1. Comme on peut le voir sur cette figure, le dispositif 2 est un appareil d'hématologie classique comprenant une seringue 40 de diamètre 1,5mm, et une seringue 42 de diamètre 16mm qui sont toutes deux reliées à l'aiguille 8. Le capteur 20 a été intégré sur l'aiguille 8, de sorte qu'il recouvre l'extrémité du tube 16 reliée à l'aiguille 8.

Le dispositif 2 de la figure 4 est adapté à réaliser un frottis, une mesure ESR ou les deux.

Pour la réalisation d'un frottis par le premier groupe 4, l'ensemble du circuit est amorcé en diluant, puis l'aiguille 8 percute le tube de sang 11 et prélève environ 75 µl avec la seringue. Lors de sa remontée, l'extérieur de l'aiguille 8 est progressivement rincée par un flux de liquide poussé d'un côté et continuellement aspiré et évacué aux déchets de l'autre.

La seringue effectue ensuite un retrait de la colonne de liquide afin de transférer l'aliquote au niveau du Y d'une vanne à pince ouverte 44 du côté de l'aiguille 8. La vanne à pince est activée pour refermer le canal en provenance de l'aiguille 8 et ouvrir le canal à destination d'une aiguille dépose-goutte 46. La seringue effectue ensuite une poussée qui permet de transférer l'échantillon jusqu'à l'aiguille dépose-goutte. Une séquence de rinçage périphérique de l'aiguille dépose-goutte puis de séchage permet de s'assurer de la qualité du front initial d'aliquote. Enfin, une goutte de quelques microlitres est déposée sur une lame 48 en effectuant une poussée de la seringue.

Pour la mesure ESR, il est procédé comme décrit plus haut en référence aux figures 1 à 3. L'ensemble du circuit est amorcé en diluant tiré d'un réservoir 44, puis une bulle d'air de quelques µL est créée en bout de l'aiguille 8. L'aiguille 8 est descendue dans le tube de sang 11 et une aliquote de 50 µL à 100 µL est prélevée par la seringue 42. L'aiguille 8 est remontée, et l'aliquote transférée au capteur 20 et la mesure optique de l'agrégation est réalisée. Enfin, le rinçage intérieur et extérieur de l'aiguille 8 dans un bac est réalisé, avec évacuation aux déchets. La mesure de blanc (I0) peut être effectuée avant le prélèvement ou en fin de rinçage. La comparaison optionnelle des mesures de blanc avant et après permettent une gestion d'exception, par exemple un contrôle du rinçage.

Les cycles frottis et ESR sont réalisés indépendamment l'un de l'autre, et font en particulier l'objet d'un prélèvement par l'aiguille 8 séparé : l'aliquote pour le frottis ne peut pas être utilisée pour la mesure ESR et inversement.

Cela permet de découpler les deux opérations sans prélèvement problématique de sang lorsque les deux mesures sont demandées. Cette indépendance rend la mesure ESR beaucoup moins gênante pour la cadence du dispositif 2 et permet une intégration à moindre coût, tant matériel qu'humain.

Le dispositif de l'invention est beaucoup plus efficace que tous les systèmes connus, qui proposent soit un module spécifique pour la mesure de la vitesse de sédimentation, en plus du module d'étalement ou de coloration, ou de ponctionner du temps de traitement CBC/DIF/RET sur un module généraliste.

En variante, le deuxième groupe 6 peut être intégré sur l'aiguille de prélèvement d'un dispositif haut de gamme qui comprend un module de prélèvement spécifique pour des mesures ultérieures d'étalement ou de coloration.

L'invention permet donc d'intégrer de manière efficace la mesure de vitesse de sédimentation aux dispositifs existants, sans impacter leurs cadences de travail ou leur architecture.

## Revendications

1. Dispositif d'étalement ou coloration et de détermination de vitesse de sédimentation, comprenant un premier groupe (4) agencé pour prélever un échantillon sanguin dans un tube et pour réaliser un frottis sur cet échantillon, **caractérisé en ce qu'**il comprend un deuxième groupe (6) agencé pour prélever un échantillon sanguin dans un tube et pour réaliser une détermination de vitesse de sédimentation, le dispositif comprenant au moins un organe de prélèvement (8) pouvant être commandé pour une opération par le premier groupe (4) et une opération par le deuxième groupe (6) pour le prélèvement d'un échantillon sanguin de sorte qu'un échantillon prélevé pour le premier groupe (4) n'est pas utilisé par le deuxième groupe (6), et qu'un échantillon prélevé pour le deuxième groupe (6) n'est pas utilisé par le premier groupe (4), le deuxième groupe (6) étant muni d'un capteur (20) comprenant une source de lumière à infrarouge (12) et un capteur optique (14) disposés sensiblement en regard l'un de l'autre autour d'un tube (16) relié à une extrémité de sortie de l'au moins un organe de prélèvement (8) de sorte que la lumière émise par la source de lumière infrarouge (12) atteint le capteur optique (14) après avoir traversé ledit tube (16), le deuxième groupe (6) étant en outre agencé pour réaliser un rinçage de l'organe de prélèvement (8) et du tube (16) entre deux déterminations de mesures de vitesse de sédimentation et le capteur optique (14) étant agencé pour réaliser une mesure de blanc après une opération de rinçage, le dispositif comprenant en outre un convertisseur (10) agencé pour recevoir une mesure de blanc et une ou plusieurs mesures de transmission lumineuse du capteur optique (14) et pour déterminer une vitesse de sédimentation à partir du rapport entre la mesure de blanc et la ou les plusieurs mesures de transmission lumineuse.

2. Dispositif selon la revendication 1, dans lequel le convertisseur (10) est agencé pour déterminer un instant de mesure de transmission lumineuse la plus basse (t3), et un instant de mesure de transmission lumineuse final (t4).

3. Dispositif selon la revendication 2, dans lequel le capteur optique (14) est agencé pour mettre en œuvre un gain maximal entre l'instant de mesure de transmission lumineuse la plus basse (t3) et l'instant de mesure de transmission lumineuse final (t4), et pour mettre en œuvre un gain minimal le reste du temps.

4. Dispositif selon la revendication 2 ou 3, dans lequel le convertisseur (10) est agencé pour calculer la vitesse de sédimentation à partir du ratio entre d'une part le rapport entre la mesure de blanc et la mesure à l'instant de mesure de transmission final (t4), et d'autre part le rapport entre la mesure de blanc et la mesure à l'instant de mesure de transmission lumineuse de transmission lumineuse la plus basse (t3).

5. Dispositif selon l'une des revendications précédentes, dans lequel le capteur optique (14) est commandé avec un gain faible avant que du sang passe dans la portion sensiblement transparente, et avec un gain fort après.

6. Dispositif selon l'une des revendications précédentes, dans lequel l'organe de prélèvement est une aiguille (8) pouvant être commandée pour le prélèvement d'un échantillon sanguin à laquelle est relié un tube (16) dans lequel est réalisée la portion sensiblement transparente (16).

7. Procédé d'étalement ou coloration et de détermination de vitesse de sédimentation **caractérisé en ce qu'**il comprend l'utilisation d'un dispositif selon l'une des revendications 1 à 6, et **en ce que** la réalisation d'un étalement ou coloration d'une part et la détermination d'une vitesse de sédimentation d'autre part comprennent le prélèvement de deux échantillons distincts.

## Patentansprüche

1. Vorrichtung zum Ausstreichen oder Kolorieren und zum Bestimmen einer Sedimentationsgeschwindigkeit, umfassend eine erste Gruppe (4), welche dazu eingerichtet ist, eine Blutprobe in einem Röhrchen zu entnehmen und einen Ausstrich dieser Probe vorzunehmen, **dadurch gekennzeichnet, dass** sie eine zweite Gruppe (6) umfasst, welche dazu eingerichtet ist, eine Blutprobe in einem Röhrchen zu entnehmen und eine Bestimmung einer Sedimentationsgeschwindigkeit vorzunehmen, wobei die Vorrichtung wenigstens ein Entnahmeelement (8) umfasst, welches für einen Betrieb durch die erste Gruppe (4) und einen Betrieb durch die zweite Gruppe (6) für die Entnahme einer Blutprobe derart gesteuert werden kann, dass eine entnommene Probe für die erste Gruppe (4) nicht von der zweiten Gruppe (6) verwendet wird und eine entnommene Probe für die zweite Gruppe (6) nicht von der ersten Gruppe (4) verwendet wird, wobei die zweite Gruppe (6) mit einem Sensor (20) bereitgestellt ist, welcher eine Infrarot-Lichtquelle (12) und einen optischen Sensor (14) umfasst, welche im Wesentlichen einander gegenüber um ein Röhrchen (16) herum angeordnet sind, welches mit einem Ausgangsende des wenigstens einen Entnahmeelements (8) derart verbunden ist, dass das durch die Infrarot-Lichtquelle (12) emittierte Licht den optischen Sensor (14) erreicht, nachdem es das Röhrchen (16) durchquert hat, wobei die zweite Gruppe (6) ferner dazu eingerichtet ist, ein Spülen des Entnahmeelements (8) und des Röhrchens (16) zwischen zwei Bestimmungen von Messwerten der Sedimentationsgeschwindigkeit vorzunehmen, und der optische Sensor (14) dazu eingerichtet ist, einen Weißmesswert nach einem Spülvorgang vorzunehmen, wobei die Vorrichtung ferner eine Konvertierungseinheit (10) umfasst, welche dazu eingerichtet ist, einen Weißmesswert und einen oder mehrere Messwerte einer Lichttransmission des optischen Sensors (14) zu erhalten und eine Sedimentationsgeschwindigkeit ausgehend von dem Verhältnis zwischen dem Weißmesswert und dem einen oder den mehreren Messwerten einer Lichttransmission zu bestimmen.

2. Vorrichtung nach Anspruch 1, wobei die Konvertierungseinheit (10) dazu eingerichtet ist, einen Zeitpunkt eines niedrigsten Messwerts der Lichttransmission (t3) und einen Zeitpunkt eines finalen Messwerts der Lichttransmission (t4) zu bestimmen.

3. Vorrichtung nach Anspruch 2, wobei der optische Sensor (14) dazu eingerichtet ist, eine maximale Verstärkung zwischen dem Zeitpunkt des niedrigsten Messwerts der Lichttransmission (t3) und dem Zeitpunkt des finalen Messwerts der Lichttransmission (t4) auszuführen und eine minimale Verstärkung den Rest der Zeit auszuführen.

4. Vorrichtung nach Anspruch 2 oder 3, wobei die Konvertierungseinheit (10) dazu eingerichtet ist, die Sedimentationsgeschwindigkeit ausgehend von dem Quotienten zwischen einerseits dem Verhältnis zwischen dem Weißmesswert und dem Messwert zu dem Zeitpunkt des finalen Messwerts der Transmission (t4) und andererseits dem Verhältnis zwischen dem Weißmesswert und dem Messwert zum Zeitpunkt des niedrigsten Messwerts der Lichttransmission (t3) zu berechnen.

5. Vorrichtung nach einem der vorhergehenden Ansprüche, wobei der optische Sensor (14) mit einer schwachen Verstärkung gesteuert wird, bevor das Blut in dem im Wesentlichen transparenten Abschnitt verläuft, und mit einer starken Verstärkung danach.

6. Vorrichtung nach einem der vorhergehenden Ansprüche, wobei das Entnahmeelement eine Nadel (8) ist, welche zur Entnahme einer Blutprobe gesteuert werden kann, mit welcher ein Röhrchen (16) verbunden ist, in welchem der im Wesentlichen transparente Abschnitt (16) gebildet ist.

7. Verfahren zum Ausstreichen oder Kolorieren und zum Bestimmen einer Sedimentationsgeschwindigkeit, **dadurch gekennzeichnet, dass** es die Verwendung einer Vorrichtung nach einem der Ansprüche 1 bis 6 umfasst und dass das Ausführen eines Ausstreichens oder Färbens einerseits und das Bestimmen einer Sedimentationsgeschwindigkeit andererseits das Entnehmen von zwei unterschiedlichen Proben umfasst.

## Claims

1. A device for spreading or colouring and determining a sedimentation rate, comprising a first group (4) arranged so as to sample a blood sample from a tube and to carry out a smear test on this sample, **characterised in that** it comprises a second group (6) arranged so as to sample a blood sample from a tube and to carry out a sedimentation rate determination, the device comprising at least one sampling member (8) which can be controlled for an operation by the first group (4) and an operation by the second group (6) for sampling a blood sample so that a sample sampled for the first group (4) is not used by the second group (6), and that a sample sampled for the second group (6) is not used by the first group (4), the second group (6) being provided with a sensor (20) comprising an infrared light source (12) and an optical sensor (14) arranged substantially opposite one another around a tube (16) connected to an output end of the at least one sampling member (8) so that the light emitted by the infrared light source (12) reaches the optical sensor (14) after having crossed said tube (16), the second group (6) being further arranged so as to carry out a rinsing of the sampling member (8) and of the tube (16) between two sedimentation rate measurement determinations and the optical sensor (14) being arranged so as to carry out a blank measurement after a rinsing operation, the device further comprising a converter (10) arranged so as to receive a blank measurement and one or more light transmission measurement(s) from the optical sensor (14) and to determine a sedimentation rate from the ratio between the blank measurement and the light transmission measurement(s).

2. The device according to claim 1, wherein the converter (10) is arranged so as to determine a time point of measurement of the lowest light transmission (t₃), and a time point of measurement of the final light transmission (t₄).

3. The device according to claim 2, wherein the optical sensor (14) is arranged so as to implement a maximum gain between the time point of measurement of the lowest light transmission (t₃) and the time point of measurement of the final light transmission (t₄), and to implement a minimum gain the rest of the time.

4. The device according to claim 2 or 3, wherein the converter (10) is arranged so as to calculate the sedimentation rate from the ratio between, on the one hand, the ratio between the blank measurement and the measurement at the time point of measurement of the final light transmission (t₄) and, on the other hand, the ratio between the blank measurement and the measurement at the time point of measurement of the lowest light transmission (t₃).

5. The device according to one of the preceding claims, wherein the optical sensor (14) is controlled with a low gain before blood passes through the substantially transparent portion, and with a high gain afterwards.

6. The device according to one of the preceding claims, wherein the sampling member is a needle (8) which can be controlled for sampling of a blood sample to which a tube (16) is connected in which the substantially transparent portion (16) is formed.

7. A method for spreading or colouring and determining a sedimentation rate **characterised in that** it comprises using a device according to one of claims 1 to 6, and **in that** carrying out a spreading or colouring on the one hand and determining a sedimentation rate on the other hand comprise sampling two distinct samples.
